# FASCICULE DE BREVET EUROPEEN

(11) **EP 0 677 506 B1**
(45) Date de publication et mention de la délivrance du brevet: **03.09.1997**
(21) Numéro de dépôt: 95400784.5
(22) Date de dépôt: 07.04.1995
(51) Int. Cl.: C07C 67/04, C07C 69/54

(54) **Procédé de préparation de l'acrylate de butyle secondaire**
Verfahren zum Herstellen von sek. Butylacrylat
Process for the preparation of secondary butyle acrylate

(30) Priorité: 15.04.1994 FR 9404525
(43) Date de publication de la demande: 18.10.1995
(73) Titulaire: ELF ATOCHEM S.A., 92800 Puteaux (FR)
(72) Inventeur: Riondel, Alain, F-57600 Forbach (FR)

(56) Documents cités:
- EP-A- 0 445 859
- WO-A-87/06244

## Description

La présente invention se rapporte aux procédés de préparation de l'acrylate de butyle secondaire par réaction, de l'acide acrylique avec le butène-1, le butène-2 ou leur mélange en présence d'un catalyseur acide.

On connaît déjà des procédés de ce genre utilisant comme catalyseur une résine échangeuse d'ions acide à groupes acides sulfoniques (US-3 037 052), de l'acide méthanesulfonique (Brevet brésilien -78 022 55), une résine échangeuse d'ions à groupes acides sulfoniques (EP-A-445 859) de texture perfectionnée et un silicotungstate de césium, de rubidium, de thallium, d'ammonium ou de potassium (JP-A-4 139 148).

On a maintenant trouvé un catalyseur qui donne une sélectivité en acrylate de butyle meilleure que tous les catalyseurs connus et qui représente le meilleur compromis activité/sélectivité.

L'invention a donc pour objet un procédé de préparation de l'acrylate de butyle secondaire de formule 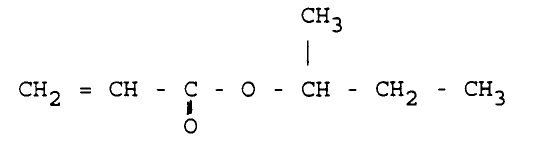 en faisant réagir de l'acide acrylique de formule 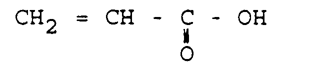 avec du butène-1 de formule

CH₂ = CH - CH₂ - CH₃

du butène-2 de formule

CH₃ - CH = CH - CH₃ ou leur mélange

en présence d'une quantité catalytique d'un catalyseur acide, caractérisé en ce qu'il consiste à utiliser, comme catalyseur, un superacide solide à base de zirconium.

On peut effectuer la réaction entre 50 et 120°C sous une pression de 5 à 30 bar avec une durée de réaction de 0,5 à 6 heures et avec un rapport du butène à l'acide acrylique 0,3 à 10 et en présence de stabilisant comme l'éther méthylique de l'hydroquinone, la phénothiazine, l'hydroquinone et leur mélanges en toutes proportions utilisé en quantité comprise entre 150 et 3000 ppm par rapport à l'acide acrylique.

Comme catalyseur, on peut utiliser notamment le produit de réaction de l'hydroxyde de zirconium et du sulfate d'ammonium.

Les exemples suivants illustrent l'invention.

### Exemple 1 : Préparation d'un superacide solide à base de zirconium

### I. Préparation du Zr(OH)₄

Dans un réacteur d'1 litre, double enveloppe, muni de
. une agitation mécanique,
. une sonde de température,
. une sonde pH,
on dissout à température ambiante 88 g de ZrOCl₂ octahydraté dans 800 ml d'eau.

On introduit lentement, toujours à température ambiante, environ 50 ml de NH₄OH à 25 % de telle façon que le pH de la solution finale soit basique et compris entre 8 et 9.

Le précipité blanc de Zr(OH)₄ obtenu est ensuite :
1) lavé à l'eau pour éliminer les ions chlorure,
2) séché à l'étuve à 100°C sous pression réduite pendant une nuit.

On obtient ainsi environ 40 g de Zr(OH)₄ sec.

### II. Sulfatation - calcination

On mélange à l'aide d'un broyeur Zr(OH)₄ et NH₄(SO₄)₂ (15 % en poids par rapport à Zr(OH)₄).

Le solide obtenu est ensuite calciné sous l'air sec à 650°C.

Après refroidissement, on obtient un solide blanc que l'on désigne par ZrSA15.

### Exemple 2 : Préparation d'acrylate de butyle secondaire

Dans un réacteur en acier inoxydable de 200 ml de capacité, muni d'un thermomètre et d'un manomètre, on charge à la température ambiante 0,9 mole (51 g de butène-1 liquide), une mole (72 g d'acide acrylique), 11 g (8,5 % en poids/charge) de ZrSA15 et 0,018 g (250 ppm/par rapport à l'acide acrylique) d'éther méthylique de l'hydroquinone servant de stabilisant. On ferme le réacteur et on l'immerge dans un bain d'huile à 150°C. On maintient le milieu réactionnel à 100°C sous agitation pendant quatre heures. La pression passe de 10 bar en début de réaction à 4 bar en fin de réaction. On refroidit le mélange réactionnel en fin de réaction jusqu'à la température ambiante. On ouvre ensuite le réacteur de façon à dégazer le butène qui n'a pas réagi.

On soumet le produit obtenu aux analyses suivantes :
- potentiométrie,
- chromatographie en phase gazeuse.

On détermine ainsi la conversion de l'acide acrylique par dosage potentiométrique, la conversion du butène-1 à partir de la masse de butène-1 dégazé et du butène-1 résiduel dissous dans le milieu par chromatographie en phase gazeuse et le rendement en acrylate de butyle secondaire par dosage en chromatographie en phase gazeuse.

Ces valeurs permettent d'obtenir la sélectivité en acrylate de butyle secondaire par rapport à l'acide acrylique et par rapport au butène-1. Les résultats obtenus sont rassemblés aux tableaux 1 et 2.

### Exemple 3 (comparatif)

On reprend l'exemple 2, si ce n'est que l'on remplace le catalyseur par une résine acide comportant des groupes sulfoniques Amberlyst 15 vendue par Rohm et Haas.

Les résultats obtenus sont consignés aux tableaux 1 et 2.

### Exemples 4 et 5 (comparatifs)

On reprend l'exemple 2, si ce n'est que l'on remplace le catalyseur respectivement par d'autres catalyseurs acides H₃PW₁₂O₄₀ et Cs₃PW₁₂O₄₀.

Les résultats obtenus apparaissent aux tableaux 1 et 2.

**Tableau 1**

| Analyse (%) en chromotographie en phase gazeuse des bruts réactionnels obtenus | | | | | | | | | |
|---|---|---|---|---|---|---|---|---|---|
| Exemple | Catalyseur | C4 | BU-2-OH | C8 | AA(a) | ABU2 | Dimère | AC8 | AC12 |
| 2 | ZrSA 15 | 10,4 | 0,25 | 1,8 | 27,5 | 57 | 1,3 | 0,13 | 0,16 |
| 3 | A 15 | 7,6 | 0,4 | 2,6 | 25,6 | 57,4 | 3,2 | 0,16 | 0,5 |
| 4 | H₃PW₁₂O₄₀ | 6,9 | 0,5 | 3,6 | 28,3 | 48,3 | 1,8 | 0,25 | 2,8 |
| 5 | Cs₃PW₁₂O₄₀ | 9,8 | 0,58 | 4 | 26,6 | 53,4 | 2,27 | 0,21 | 2,1 |
| (a) Dosé en potentiométrie C4: Butène BU 2-OH: Butanol secondaire C8: Dibutène A C8: Acrylate en C8 A C12: Acrylate en C12 | | | | | | | | | |

**Tableau 2**

| Synthèse de l'ABU 2 - Résultats | | | | | | |
|---|---|---|---|---|---|---|
| Exemple | Bilan matière | % CAA(%) | CC4(%) | R ABU-2 (%) | S/C4 (%) | S/AA (%) |
| 2 | 97,7 | 60 | 63 | 59 | 93,6 | 98,3 |
| 3 | 98 | 67 | 80 | 61,5 | 76,2 | 91,8 |
| 4 | 100 | 53 | 80 | 52 | 65 | 99,6 |
| 5 | 100 | 56 | 78 | 53 | 68 | 94,6 |
| C : conversion R : rendement S : sélectivité | | | | | | |

Les activités des catalyseurs testés se classent dans l'ordre suivant :
Amberlyst ≃ Zr SA 15 (OP 135) > CS₃PW₁₂O₄₀, H₃PW₁₂O₄₀

En sélectivité en ABU-2/C4, l'ordre devient :
ZrSA15 > Amberlyst > Cs₃PW₁₂O₄₀, H₃PW₁₂O₄₀

Il ressort donc de ces essais que le meilleur compromis activité/sélectivité est représenté par l'utilisation de ZrSA15.

## Revendications

1. Procédé de préparation de l'acrylate de butyle secondaire de formule 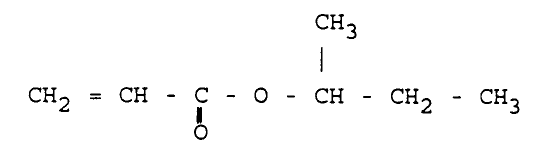 en faisant réagir de l'acide acrylique de formule 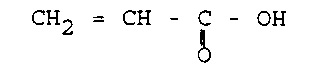 avec du butène-1 de formule
CH₂ = CH - CH₂ - CH₃
du butène-2 de formule
CH₃ - CH = CH - CH₃ ou leur mélange
en présence d'une quantité catalytique d'un catalyseur acide,
caractérisé en ce qu'il consiste à utiliser, comme catalyseur, un superacide solide à base de zirconium.

2. Procédé suivant la revendication 1, caractérisé en ce qu'il consiste à effectuer la réaction entre 50 et 120°C sous une pression de 5 à 30 bar, avec une durée de réaction de 0,5 à 6 heures, et avec un rapport de butène-1 à l'acide acrylique de 0,3 à 10 et en présence de stabilisant comme l'éther méthylique de l'hydroquinone, la phénothiazine, l'hydroquinone et leurs mélanges en toutes proportions utilisé en quantité comprise entre 150 et 3000 ppm par rapport à l'acide acrylique.

3. Procédé suivant la revendication 1 ou 2, caractérisé en ce qu'il consiste à utiliser comme catalyseur le produit de réaction de l'hydroxyde de zirconium et du sulfate d'ammonium.

4. Le produit de réaction de l'hydroxyde de zirconium et du sulfate d'ammonium.

## Claims

1. Process for preparing sec-butyl acrylate of formula 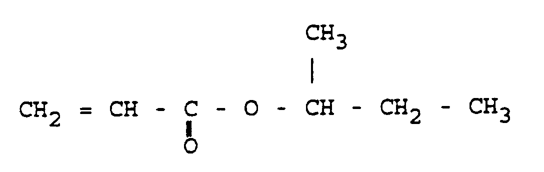 by reacting acrylic acid of formula 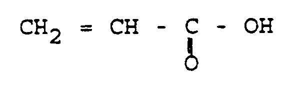 with 1-butene of formula
CH₂ = CH - CH₂ - CH₃
2-butene of formula
CH₃ - CH = CH - CH₃ or a mixture thereof
in the presence of a catalytic amount of an acid catalyst, characterized in that it consists in using, as catalyst, a zirconium-based solid superacid.

2. Process according to Claim 1, characterized in that it consists in carrying out the reaction between 50 and 120°C at a pressure of 5 to 30 bar with a reaction time of 0.5 to 6 hours and with a 0.3 to 10 butene-to-acrylic acid ratio and in the presence of a stabilizing agent such as hydroquinone methyl ether, phenothiazine, hydroquinone and mixtures thereof in all proportions, which is used in an amount between 150 and 3000 ppm relative to the acrylic acid.

3. Process according to Claim 1 or 2, characterized in that it consists in using as catalyst the product of reaction of zirconium hydroxide and ammonium sulphate.

4. The product of reaction of zirconium hydroxide and ammonium sulphate.

## Patentansprüche

1. Verfahren zur Herstellung von sek.-Butylacrylat der Formel 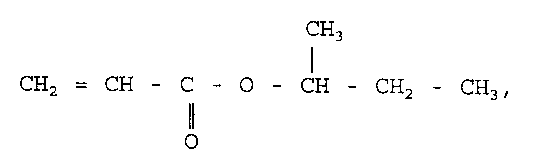 wobei Acrylsäure der Formel 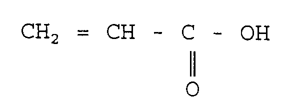 mit 1-Buten der Formel
CH₂ = CH - CH₂ - CH₃,
2-Buten der Formel
CH₃ - CH = CH - CH₃
oder der Gemischen
in Gegenwart eine katalytischen Menge eines sauren Katalysators umgesetzt wird,
dadurch gekennzeichnet, daß
als Katalysator eine feste Supersäure auf der Basis von Zirconium verwendet wird.

2. Verfahren nach Anspruch 1, dadurch gekennzeichnet, daß die Umsetzung bei einer Temperatur im Bereich von 50 bis 120 °C unter einem Druck von 5 bis 30 bar während einer Zeitdauer der Umsetzung von 0,5 bis 6 h bei einem Verhältnis von 1-Buten zu Acrylsäure von 0,3 bis 10 in Gegenwart eines Stabilisierungsmittels, wie Hydrochinonmethylether, Phenothiazin, Hydrochinon und deren Gemischen in beliebigen Verhältnissen, in einem Mengenanteil im Bereich von 150 bis 3000 ppm, bezogen auf die Acrylsäure, durchgeführt wird.

3. Verfahren nach Anspruch 1 oder 2, dadurch gekennzeichnet, daß als Katalysator das Reaktionsprodukt von Zirconiumhydroxid und Ammoniumsulfat verwendet wird.

4. Reaktionsprodukt von Zirconiumhydroxid und Ammoniumsulfat.
